# EUROPEAN PATENT SPECIFICATION

(11) **EP 3 463 309 B1**
(45) Date of publication and mention of the grant of the patent: **17.06.2020**
(21) Application number: 17726262.3
(22) Date of filing: 24.05.2017
(51) Int. Cl.: A61K 9/20, A61K 9/24, A61K 31/4184, A61K 31/4422, A61K 31/495

(54) **FIXED DOSE COMBINATION OF TELMISARTAN, HYDROCHLOROTHIAZIDE AND AMLODIPINE**
FESTDOSEN-KOMBINATION VON TELMISARTAN, HYDROCHLOROTHIAZID UND AMLODIPIN
COMBINAISON DE DOSE FIXE DE TELMISARTAN, DE L'HYDROCHLOROTHIAZIDE ET DE L'AMLODIPINE

(30) Priority: 30.05.2016 EP 16171934
(43) Date of publication of application: 10.04.2019
(73) Proprietor: Boehringer Ingelheim International GmbH, 55216 Ingelheim am Rhein (DE)
(72) Inventor: NAKATANI, Manabu, 55216 INGELHEIM AM RHEIN (DE); SEKI, Noriko, 55216 INGELHEIM AM RHEIN (DE); YOKOYAMA, Kazutoshi, 55216 INGELHEIM AM RHEIN (DE)
(74) Representative: Simon, Elke Anna Maria
(86) International application number: PCT/EP2017/062516
(87) International publication number: WO 2017/207375

(56) References cited:
- WO-A1-03/059327
- WO-A1-2006/048208
- CN-A- 101 690 725
- US-A1- 2010 003 321

## Description

### Field of Invention

The present invention relates to a pharmaceutical tablet with a first layer comprising telmisartan, and a second layer comprising the diuretic hydrochlorothiazide, the calcium channel blocker amlodipine, 10-60% mannitol, 10-50% microcrystalline cellulose and 5-25% corn starch. Also provided is a method of producing such a pharmaceutical tablet.

### Background of the invention

Telmisartan is an angiotensin II receptor antagonist used for the treatment of hypertension and other medical indications as disclosed in EP-A-502314. Its chemical name is 4'-[2-n-propyl-4-methyl-6-(1-methylbenzimidazol-2-yl)-benzimidazol-1-ylmethyl]-biphenyl-2-carboxylic acid having the following structure:

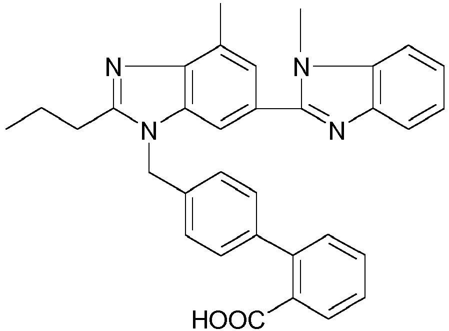

Telmisartan is manufactured and supplied in the free acid form, which is characterized by poor solubility in aqueous systems with a pH value between pH 1 to 7, i.e. the physiological pH range of the gastro-intestinal tract. As disclosed in WO 00/43370, crystalline telmisartan exists in two polymorphic forms having different melting points. Under the influence of heat and humidity, the lower melting polymorph B transforms irreversibly into the higher melting polymorph A.

To achieve synergistic therapeutic efficacy in the treatment of hypertension anti-hypertensive diuretics are occasionally combined with anti-hypertensive agents acting on the basis of a different mode of action. A preferred diuretic is the thiazide diuretic hydrochlorothiazide (HCTZ).The chemical name of HCTZ is 6-chloro-3,4-dihydro-2H-1,2,4-benzothiadiazine-7-sulfonamide-1,1-dioxide having the following structure

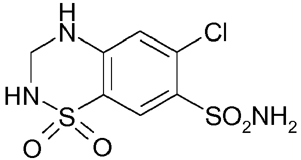

Amlodipine was first disclosed in EP-A-89167 and belongs to the group of calcium channel blockers also called "calcium antagonists" or "calcium blockers". These medications decrease the heart's pumping strength and relax blood vessels. They are used to treat high blood pressure, angina (chest pain or discomfort caused by reduced blood supply to the heart muscle) and some arrhythmias. Amlodipine's chemical name is 3-ethyl-5-methyl-2-(2-aminoethoxymethyl)-4-(2-chlorophenyl)-1,4-dihydro-6-methyl-3,5-pyridinedicarboxylate (C₂₀H₂₅ClN₂O₅, molar mass 408,88) having the following stucture:

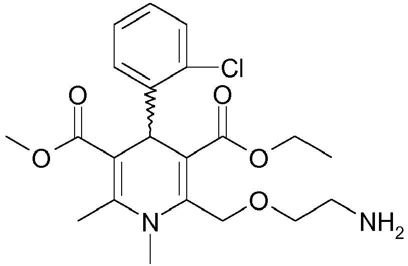

Pharmaceutically it is is used as the maleate (C₂₄H₂₉ClN₂O₉; molar mass 524,96), benzenesulfonate or besylate (C₂₆H₃₁ClN₂O₈S; molar mass 567,10; EP-A-244944) and mesylate salt (C₂₁H₂₆ClN₂O₈S; molar mass 502,01).

### Objects of the invention

WO03/059327 discloses a pharmaceutical tablet comprising a first layer comprising substantially amorphous telmisartan in a dissolving tablet matrix, and a second layer comprising a diuretic in a disintegrating tablet matrix. WO2006/048208 discloses a pharmaceutical tablet comprising a first layer comprising telmisartan in a dissolving tablet matrix and a second layer comprising amlodipine in a disintegrating or eroding tablet matrix.

To improve treatment of hypertension in patients whose hypertension is not adequately controllled by a combination of telmisartan and HCTZ or telmisartan and amlodipine, the inventors attempted to provide a two-layered combination tablet comprising telmisartan, HCTZ and amlodipine. Unexpectedly, friction by dice (die friction) and peeling of the two layers (delamination) were perceived. These problems were not perceived for the tablets only combining telmisartan and HCTZ, or telmisartan and amlodipine. Thus, combining HCTZ and amlodipine in the same layer resulted in an unfavourable impact on drug stability.

Therefore, one of the objects of the invention is to solve the tableting problems encountered in connection with a pharmaceutical fixed dose tablet combining telmisartan, HCTZ and amlodipine.

Another object of the invention is to provide a method for producing such a pharmaceutical fixed dose tablet comprising telmisartan, HCTZ and amlodipine achieving drug stability combined with suitable drug dissolution.

### Summary of the invention

Described is a convenient combination of excipients for a pharmaceutical fixed dose tablet comprising telmisartan, HCTZ and amlodipine, which ensures acceptable drug stability and dissolution.

### Description of the preferred embodiments

A pharmaceutical tablet according to the present invention consists of a first layer comprising telmisartan, preferably in amorphous form, and a second layer comprising amlodipine and hydrochlorothiazide (HCTZ), 10-60% mannitol, 10-50% microcrystalline cellulose and 5-25% corn starch.

The active ingredient telmisartan is an acid, but pharmaceutically acceptable salts such as the sodium salt may also be used. Telmisartan is conveniently dissolved and transformed into an amorphous form but it is not required to use amorphous telmisartan. Telmisartan can also be used in crystalline and partially crystalline form. Amorphous telmisartan can be produced by methods known to those skilled in the art, for instance, by freeze or spray drying of aqueous solutions, coating of carrier particles in a fluidized bed, or solvent deposition on sugar pellets or other carriers. Preferably, at least 90% substantially amorphous telmisartan as determined by X-ray powder diffraction is prepared by the method described in EP-1854454-B1.

A bilayer tablet according to the present invention comprises 10 to 160 mg, preferably 40 to 80 mg, of telmisartan in the first layer; and 2.5 to 10 mg, of amlodipine, and 10 to 15 mg, of HCTZ. Preferred dose strengths of telmisartan are 40 mg and 80 mg; preferred dose strengths of amlodipine are 5 mg and 10 mg; a preferred dose strength of HCTZ is 12.5 mg Presently preferred forms are bilayer tablets comprise 40/5/12.5 mg, 40/10/12.5 mg, 80/5/12.5 mg, and 80/10/12.5 mg of telmisartan, amlodipine, and HCTZ, respectively.

The first layer preferably contains telmisartan in substantially amorphous form as determined by X-ray powder diffraction dispersed in a dissolving tablet matrix having fast dissolution characteristics. The telmisartan layer comprises a basic agent, a water-soluble diluent and, optionally, other excipients and adjuvants. Specific examples of suitable basic agents are alkali metal hydroxides such as NaOH and KOH; basic amino acids such as arginine and lysine; and meglumine (N-methyl-0-glucamine). NaOH and meglumine are preferred basic agents.

Specific examples of suitable water-soluble diluents are carbohydrates such as monosaccharides like glucose; oligosaccharides like sucrose, anhydrous lactose and lactose monohydrate; and sugar alcohols like mannitol, sorbitol, erythritol, and xylitol. Mannitol is a preferred diluent. The other excipients and/or adjuvants are, for instance, selected from binders, carriers, fillers, lubricants, flow control agents, crystallization retarders, solubilizers, coloring agents, pH control agents, surfactants and emulsifiers, specific examples of which are given below in connection with the second tablet layer composition. The excipients and/or adjuvants for the first tablet layer composition are preferably chosen such that a fast dissolving tablet matrix is obtained.

Generally, the first layer composition comprises 3 to 50 wt.%, preferably 5 to 35 wt.%, of telmisartan; 0.25 to 20 wt.%, preferably 0.40 to 15 wt.%, of basic agent; and 30 to 95 wt.%, preferably 60 to 80 wt.% of water-soluble diluent (filler). Other (optional) constituents may, for instance, be chosen from one or more of the following excipients and/or adjuvants in the amounts indicated: 10 to 30 wt.%, preferably 15 to 25 wt.%, of binders, carriers and fillers, thereby replacing the water-soluble diluent; 0.1 to 5 wt.%, preferably 0.5 to 3 wt.%, of lubricants; 0.1 to 5 wt.%, preferably 0.3 to 2 wt.%, of flow control agents; 1 to 10 wt.%, preferably 2 to 8 wt.%, of crystallization retarders; 1 to 10 wt.%, preferably 2 to 8 wt.%, of solubilizers; 0.05 to 1.5 wt.%, preferably 0.1 to 0.8 wt.%, of coloring agents; 0.5 to 10 wt.%, preferably 2 to 8 wt.%, of pH control agents; 0.01 to 5 wt.%, preferably 0.05 to 1 wt.%, of surfactants and emulsifiers. The surfactants and emulsifiers may be ionic or non-ionic, the latter being preferred. Examples of surfactants and emulsifiers are poloxamers or pluronics, polyethylene glycols, polyethylene glycol monostearate, polysorbates, sodium lauryl sulfate, polyethoxylated and hydrogenated castor oil etc.

With regard to the poloxamers or pluronics suitable as non-ionic surfactants and emulsifiers reference is made to The Merck Index, 12th edition, 1996 herewith incorporated by reference. Suitable poloxamers suppress variation of dissolution and have an average molecular weight of about 2000 to 12000 Dalton, preferably 4000 to 10000 Dalton, even more preferred 6000 to 10000 Dalton and most preferred 8000 to 9000 Dalton. Specific poloxamers are poloxamer 182LF, poloxamer 331 and poloxamer 188.

The second layer composition comprises amlodipine and HCTZ dispersed in a disintegrating or eroding tablet matrix having instant release (fast dissolution) characteristics. Excipients and/or adjuvants for the second layer composition are preferably chosen such that a neutral, disintegrating or eroding tablet matrix is obtained. As solvent for the granulation liquid, which, as a volatile component, does not remain in the final product, methanol, ethanol, isopropyl alcohol or purified water can be used; preferred solvent is purified water.

The disintegrating or eroding matrix can comprise one or more fillers, a disintegrant, a lubricant and, optionally a flow control agent, a binder or polymer, other excipients and adjuvants. Fillers for the second layer can be selected from the group consisting of cornstarch, pregelatinized starch, microcrystalline cellulose, cellulose, mannitol, erythritol, lactose monohydrate, dibasic calcium phosphate anhydrous, sorbitol, and xylitol. Preferred fillers are mannitol, cornstarch, and microcrystalline cellulose. Disintegrants can be selected from the group consisting of cornstarch, pregelatinized starch, low-substituted hydroxypropylcellulose (L-HPC), croscarmellose sodium, sodium starch glycolate, crospovidone, and microcrystalline cellulose. Particularly preferred is the disintegrant cornstarch.

A particularly preferred binder is microcrystalline cellulose. Preferred lubricants can be sodium stearyl fumarate and magnesium stearate. Other excipients and adjuvants, which can be used, are for example flow control agents such as colloidal silicon dioxide or talc, coloring agents including dyes and pigments such as iron oxides.

Generally, the second tablet layer composition comprises
2 to 15 wt.%, preferably 3 to 8 wt.% of amlodipine;
5 to 15 wt.%, preferably 8 to 10 wt.%, of HCTZ; and
70 to 95 wt.%, preferably 80 to 90 wt.% of fillers.

In particular the second layer composition comprises
10-60%, preferably 20-50% mannitol,
10-50%, preferably 20-40% microcrystalline cellulose and
5-25%, preferalby 10-20% corn starch.

Other excipients and/or adjuvants can be selected from binders (0 to 7 wt. %, preferably 1 to 5 wt. %), disintegrants (0 to 10 wt. %, preferably 1 to 5 wt. %), lubricants (0.25 to 3 wt. %, preferably 0.5 to 2 wt. %), flow control agents (0 to 3 wt. %, preferably 0 to 2 wt. %) and coloring agents (0.05 to 3 wt. %, preferably 0.1 to 1 wt. %). The excipients and/or adjuvants for the second layer composition are preferably chosen such that a neutral, disintegrating or eroding tablet matrix is obtained. As solvent for the granulation liquid, which, as a volatile component, does not remain in the final product, methanol, ethanol, isopropyl alcohol or purified water can be used; preferred solvent is purified water.

For preparing a bilayer tablet according to the invention, the first and the second layer compositions are compressed in a bilayer tablet press, e.g. a high-speed rotary press in a bilayer tabletting mode. However, care should be taken not to employ an excessive compression force for the first tablet layer. Preferably, the ratio of the compression force applied during compression of the first tablet layer to the compression force applied during compression of both the first and second tablet layers is in the range of from 1:10 to 1:2. For instance, the first layer may be compressed at moderate force of 4 to 8 kN, whereas the main compression of first plus second layer is performed at a force of 10 to 20 kN. Adequate bond formation between the two layers is achieved by distance attraction forces (intermolecular forces) and mechanical interlocking between the particles.

The bilayer tablets obtained release the active ingredients rapidly and in a largely pH-independent fashion, with complete release occurring within less than 60 min and release of the major fraction (more than 60%) occurring within less than 15 min. In accordance with the present invention, an increased dissolution rate of the active ingredients and, in particular, of telmisartan is achieved. Normally, at least or more than 70% and typically at least or more than 90% of the drug load are dissolved after 30 min using the paddle method dissolution test conditions at 50 rpm of the Japanese Pharmacopeia at pH 6.8 . And Normally, at least 50% and typically at least 70% of the drug load are dissolved after 60 min using the paddle method dissolution test conditions at 50 rpm at pH 1.2.

To provide a first tablet layer composition an aqueous alkaline solution of telmisartan is prepared by dissolving the active ingredient in purified water with the help of one or more basic agents like sodium hydroxide and meglumine. Optionally, a solubilizer and/or a recrystallization retarder may be added. The dry matter content of the starting aqueous solution is generally 10 to 40 wt.%, preferably 20 to 30 wt.%. The water-soluble diluent is generally employed in an amount of 30 to 95 wt.%, preferably 60 to 80 wt.%, based on the weight of the first tablet layer composition. The lubricant is generally added to the premix in an amount of 0.1 to 5 wt.%, preferably 0.3 to 2 wt.%, based on the weight of the first layer composition.

To provide the first layer composition comprising telmisartan, several different manufacturing methods can be used, for example, fluid-bed granulation, spray-drying or extrusion with an extruder.

A preferred method of producing the bilayer tablet according to the invention comprises the following steps of providing the first layer composition by a fluid-bed granulation method:
a) preparing an aqueous solution of telmisartan, at least one basic agent and, optionally, a solubilizer and/or a crystallization retarder;
b) spraying said aqueous solution on a water-soluble diluent in a fluid-bed granulator to obtain a premix;
c) mixing said premix with a flow control agent and a lubricant to obtain a final blend for the first layer;
d) optionally, adding other excipients and/or adjuvants in any of steps a) to d);

To provide a second layer composition comprising amlodipine and HCTZ as active ingredients,
10 to 60 wt.%, preferably 20 to 50 wt.% of mannitol,
10 to 50 wt.%, preferably 20 to 40 wt.% of microcrystalline cellulose, and
5 to 25 wt.%, preferably 10 to 20 wt.% of cornstarch are used as fillers.

Optionally 2 to 8 wt.%, preferably 4 to 6 wt.% of hydroxypropylcellulose is used as a binder.

Magnesium stearate can be added as a lubricant to the premix in an amount of 0.1 to 5 wt.%, preferably 0.3 to 2 wt.%, based on the weight of the second layer composition.

Different manufacturing methods can be used to manufacture a second tablet layer composition comprising amlodipine and HCTZ, for example direct compression, fluid-bed granulation, wet high-shear granulation or roller compaction processes. A preferred method of producing the second layer composition by the fluid-bed granulation method comprises:
a) preparing an aqueous solution of hydroxypropylcellulose as granulation liquid;
b) blending HCTZ and amlodipine with mannitol, microcrystalline cellulose, and cornstarch in the chamber of a fluid-bed granulator;
c) granulating said blend by spraying the granulation liquid in the chamber of the fluid-bed granulator to obtain a premix of dry granulate;
d) optionally dry screening the premix of dry granulate through a screen in order to segregate cohesive particles and to improve content uniformity; and
e) blending the premix of screened premix of dry granulate with magnesium stearate as a lubricant in a blender in order to obtain the final blend ready-for-compression

For preparing a bilayer tablet according to the invention, the first and second layer compositions are compressed in a bilayer tablet press, e.g. a high-speed rotary press in a bilayer tabletting mode. However, care should be taken not to employ an excessive compression force for the first tablet layer. Preferably, the ratio of the compression force applied during compression of the first tablet layer to the compression force applied during compression of both the first and second tablet layers is in the range of from 1:10 to 1:2. For instance, the first layer may be compressed at moderate force of 4 to 8 kN, whereas the main compression of first plus second layer is performed at a force of 10 to 20 kN. Adequate bond formation between the two layers is achieved by intermolecular distance attraction forces and mechanical interlocking between the particles.

Optional an appropriate external lubricant spray system for the dies and punches can be used during manufacturing of tablets in order to improve lubrication. In order to avoid any cross-contamination between the tablet layers (which could lead to decomposition of amlodipine and/or HCTZ), any granulate residues have to be carefully removed during tabletting by intense suction of the die table within the tableting chamber.

Optionally, resultant bilayer tablets can be film-coated with a coating suspension composed of hypromellose, Macrogol 6000 (alternatively Macrogol 400 or Macrogol 4000) using a conventional coating pan.

The following, non-limiting **Examples** are given to further illustrate the present invention:

### Example 1: Bilayer tablets of Telmisartan 80 mg/ Amlodipine 5 mg/ HCTZ 12.5 mg

| **Ingredients** | **mg per tablet** | **% of 1^{st} layer** | **% of 2^{nd} layer** | **% of film-coat** |
|---|---|---|---|---|
| Telmisartan | 80.00 | 23.53 | - | - |
| Meglumine | 80.00 | 23.53 | - | - |
| Poloxamer 188 | 16.00 | 4.71 | - | - |
| D-mannitol | 159.80 | 47.00 | - | - |
| Colloidal silicon dioxide | 0.20 | 0.06 | - | - |
| Magnesium stearate | 4.00 | 1.18 | - | - |
| Purified water* | * | * | - | - |
| **Sub-total** | **340.00** | **100.00** | - | - |
| | | | | |
| Amlodipine besilate (as Amlodipine) | 6.93 (5.00) | | 4.95 | - |
| HCTZ | 12.50 | - | 8.93 | - |
| D-mannitol | 49.57 | - | 35.41 | - |
| Microcrystalline cellulose | 42.00 | - | 30.00 | |
| Cornstarch | 21.00 | - | 15.00 | - |
| Hydroxypropylcellulose | 7.00 | | 5.00 | |
| Magnesium stearate | 1.00 | | 0.71 | |
| Purified water* | * | - | * | - |
| **Sub-total** | **140.00** | **-** | **100.00** | - |
| | | | | |
| Hypromellose | 4.80 | - | - | 48.00 |
| Macrogol 6000 | 1.40 | - | - | 14.00 |
| Talc | 2.00 | - | - | 20.00 |
| Titanium oxide | 1.80 | - | - | 18.00 |
| Iron oxide yellow | Trace | - | - | Trace |
| Iron oxide red | Trace | | - | Trace |
| Purified water* | * | - | - | * |
| **Sub-total** | **10.00** | **-** | **-** | **100.00** |
| **TOTAL** | **490.00** | - | - | - |

| | | | | |
|---|---|---|---|---|
| * Volatile component, does not remain in final product | | | | |

### Reference Examples 1 and 2:

Telmisartan 80 mg/ Amlodipine 5 mg/ HCTZ 12.5 mg bilayer tablets

| | **Reference 1** | | **Reference 2** | |
|---|---|---|---|---|
| **Ingredients** | **mg per tablet** | **% per layer** | **mg per tablet** | **% per layer** |
| Telmisartan | 80.00 | 23.53 | 80.00 | 23.53 |
| Meglumine | 80.00 | 23.53 | 80.00 | 23.53 |
| Poloxamer 188 | 16.00 | 4.71 | 16.00 | 4.71 |
| D-mannitol | 159.80 | 47.00 | 159.80 | 47.00 |
| Colloidal silicon dioxide | 0.20 | 0.06 | 0.20 | 0.06 |
| Magnesium stearate | 4.00 | 1.18 | 4.00 | 1.18 |
| Purified water* | * | * | * | * |
| Sub-total | 340.00 | 100.00 | 340.00 | 100.00 |
| Amlodipine besilate (as Amlodipine) | 6.93 (5.00) | 4.95 | 6.93 (5.00) | 4.95 |
| HCTZ | 12.50 | 8.93 | 12.50 | 8.93 |
| D-mannitol | 90.87 | 64.91 | 69.87 | 49.91 |
| Microcrystalline cellulose | 21.00 | 15.00 | --- | --- |
| Cornstarch | --- | --- | 42.00 | 30.00 |
| Hydroxypropylcellulose | 7.00 | 5.00 | 7.00 | 5.00 |
| Colloidal silicon dioxide | 0.70 | 0.50 | 0.70 | 0.50 |
| Magnesium stearate | 1.00 | 0.71 | 1.00 | 0.71 |
| Purified water* | * | * | * | * |
| Sub-total | 140.00 | 100.00 | 140.00 | 100.00 |
| Hypromellose | 4.80 | 48.00 | 4.80 | 48.00 |
| Macrogol 6000 | 1.40 | 14.00 | 1.40 | 14.00 |
| Talc | 2.00 | 20.00 | 2.00 | 20.00 |
| Titanium oxide | 1.80 | 18.00 | 1.80 | 18.00 |
| Iron oxide yellow | Trace | Trace | Trace | Trace |
| Iron oxide red | Trace | Trace | Trace | Trace |
| Purified water* | * | * | * | * |
| **Sub-total** | **10.00** | **100.00** | **10.00** | **100.00** |
| **Total** | **490.00** | - | **490.00** | - |

| | | | | |
|---|---|---|---|---|
| * Volatile component, does not remain in final product | | | | |

### Comparison of Example 1 with Reference examples 1 and 2

| **Checking items** | **Example 1** | **Reference 1** | **Reference 2** |
|---|---|---|---|
| Release of telmisartan from first layer | Good | Good | Good |
| Chemical stability of telmisartan | Good | Good | Good |
| Chemical stability of amlodipine | Good | Poor | Good |
| Chemical stability of HCTZ | Good | Good | Good |
| Tabletting trouble (i.e. die friction or binding) during compression into bilayer tablets | No problem | Severe die friction and binding | Severe die friction and binding |

## Claims

1. A pharmaceutical tablet with a first layer comprising telmisartan, and a second layer comprising hydrochlorothiazide and amlodipine, wherein the second layer comprises
10-60% mannitol,
10-50% microcrystalline cellulose and
5-25% corn starch.

2. The pharmaceutical tablet of claim 1, wherein the second layer comprises is 20-50% mannitol.

3. The pharmaceutical tablet as claimed in claim 2, wherein the second tablet layer comprises 20-40% microcrystalline cellulose .

4. The pharmaceutical tablet as claimed in claim 1, wherein the second tablet layer comprises 10-20% corn starch.

5. The pharmaceutical tablet as claimed in claim 2, wherein the second tablet layer comprises 10-20% corn starch.

6. The pharmaceutical tablet as claimed in claim 3, wherein the second tablet layer comprises 10-20% corn starch.

7. The pharmaceutical tablet as claimed in claim 1, wherein more than 50% of telmisartan is dissolved 60 minutes after the start of a dissolution test at pH 1.2 in the Dissolution test of the Japanese Pharmacopeia, and
more than 70% of telmisartan is dissolved 30 minutes after the start of a dissolution test at pH 6.8 in the Dissolution test of the Japanese Pharmacopeia.

8. The pharmaceutical tablet as claimed in claim 2, wherein more than 50% of telmisartan is dissolved 60 minutes after the start of a dissolution test at pH 1.2 in the Dissolution test of the Japanese Pharmacopeia, and
more than 70% of telmisartan is dissolved 30 minutes after the start of a dissolution test at pH 6.8 in the Dissolution test of the Japanese Pharmacopeia.

9. The pharmaceutical tablet as claimed in claim 3, wherein more than 50% of telmisartan is dissolved 60 minutes after the start of a dissolution test at pH 1.2 in the Dissolution test of the Japanese Pharmacopeia, and
more than 70% of telmisartan is dissolved 30 minutes after the start of a dissolution test at pH 6.8 in the Dissolution test of the Japanese Pharmacopeia.

10. The pharmaceutical tablet as claimed in claim 3, wherein more than 50% of telmisartan is dissolved 60 minutes after the start of a dissolution test at pH 1.2 in the Dissolution test of the Japanese Pharmacopeia, and
more than 70% of telmisartan is dissolved 30 minutes after the start of a dissolution test at pH 6.8 in the Dissolution test of the Japanese Pharmacopeia.

11. The pharmaceutical tablet as claimed in claim 1, wherein
more than 70% of telmisartan is dissolved 60 minutes after the start of dissolution test at pH 1.2 in the Dissolution test of the Japanese Pharmacopeia, and
more than 90% of telmisartan is dissolved 30 minutes after the start of dissolution test at pH 6.8 in the Dissolution test of the Japanese Pharmacopeia.

12. The pharmaceutical tablet as claimed in claim 2, wherein
more than 70% of telmisartan is dissolved 60 minutes after the start of dissolution test at pH 1.2 in the Dissolution test of the Japanese Pharmacopeia, and
more than 90% of telmisartan is dissolved 30 minutes after the start of dissolution test at pH 6.8 in the Dissolution test of the Japanese Pharmacopeia.

13. The pharmaceutical tablet as claimed in claim 3, wherein
more than 70% of telmisartan is dissolved 60 minutes after the start of dissolution test at pH 1.2 in the Dissolution test of the Japanese Pharmacopeia, and
more than 90% of telmisartan is dissolved 30 minutes after the start of dissolution test at pH 6.8 in the Dissolution test of the Japanese Pharmacopeia.

14. A method of producing a pharmaceutical tablet comprising a first layer containing telmisartan, and a second layer containing hydrochlorothiazide and amlodipine, and 10-60% mannitol, 10-50% microcrystalline cellulose and 5-25% corn starch.

## Patentansprüche

1. Pharmazeutische Tablette mit einer ersten Schicht, umfassend Telmisartan, und einer zweiten Schicht, umfassend Hydrochlorothiazid und Amlodipin, wobei die zweite Schicht umfasst:
10-60% Mannitol,
10-50% mikrokristalline Cellulose und
5-25% Maisstärke.

2. Pharmazeutische Tablette von Anspruch 1, wobei die zweite Schicht 20-50% Mannitol umfasst.

3. Pharmazeutische Tablette nach Anspruch 2, wobei die zweite Tablettenschicht 20-40% mikrokristalline Cellulose umfasst.

4. Pharmazeutische Tablette nach Anspruch 1, wobei die zweite Tablettenschicht 10-20% Maisstärke umfasst.

5. Pharmazeutische Tablette nach Anspruch 2, wobei die zweite Tablettenschicht 10-20% Maisstärke umfasst.

6. Pharmazeutische Tablette nach Anspruch 3, wobei die zweite Tablettenschicht 10-20% Maisstärke umfasst.

7. Pharmazeutische Tablette nach Anspruch 1, wobei
mehr als 50% des Telmisartans 60 Minuten nach dem Beginn eines Auflösungstests bei pH 1,2 in dem Auflösungstest der Japanischen Pharmacopeia gelöst sind, und
mehr als 70% des Telmisartans 30 Minuten nach dem Beginn eines Auflösungstests bei pH 6,8 in dem Auflösungstest der Japanischen Pharmacopeia gelöst sind.

8. Pharmazeutische Tablette nach Anspruch 2, wobei
mehr als 50% des Telmisartans 60 Minuten nach dem Beginn eines Auflösungstests bei pH 1,2 in dem Auflösungstest der Japanischen Pharmacopeia gelöst sind, und
mehr als 70% des Telmisartans 30 Minuten nach dem Beginn eines Auflösungstests bei pH 6,8 in dem Auflösungstest der Japanischen Pharmacopeia gelöst sind.

9. Pharmazeutische Tablette nach Anspruch 3, wobei
mehr als 50% des Telmisartans 60 Minuten nach dem Beginn eines Auflösungstests bei pH 1,2 in dem Auflösungstest der Japanischen Pharmacopeia gelöst sind, und
mehr als 70% des Telmisartans 30 Minuten nach dem Beginn eines Auflösungstests bei pH 6,8 in dem Auflösungstest der Japanischen Pharmacopeia gelöst sind.

10. Pharmazeutische Tablette nach Anspruch 3, wobei
mehr als 50% des Telmisartans 60 Minuten nach dem Beginn eines Auflösungstests bei pH 1,2 in dem Auflösungstest der Japanischen Pharmacopeia gelöst sind, und
mehr als 70% des Telmisartans 30 Minuten nach dem Beginn eines Auflösungstests bei pH 6,8 in dem Auflösungstest der Japanischen Pharmacopeia gelöst sind.

11. Pharmazeutische Tablette nach Anspruch 1, wobei
mehr als 70% des Telmisartans 60 Minuten nach dem Beginn eines Auflösungstests bei pH 1,2 in dem Auflösungstest der Japanischen Pharmacopeia gelöst sind, und
mehr als 90% des Telmisartans 30 Minuten nach dem Beginn eines Auflösungstests bei pH 6,8 in dem Auflösungstest der Japanischen Pharmacopeia gelöst sind.

12. Pharmazeutische Tablette nach Anspruch 2, wobei
mehr als 70% des Telmisartans 60 Minuten nach dem Beginn eines Auflösungstests bei pH 1,2 in dem Auflösungstest der Japanischen Pharmacopeia gelöst sind, und
mehr als 90% des Telmisartans 30 Minuten nach dem Beginn eines Auflösungstests bei pH 6,8 in dem Auflösungstest der Japanischen Pharmacopeia gelöst sind.

13. Pharmazeutische Tablette nach Anspruch 3, wobei
mehr als 70% des Telmisartans 60 Minuten nach dem Beginn eines Auflösungstests bei pH 1,2 in dem Auflösungstest der Japanischen Pharmacopeia gelöst sind, und
mehr als 90% des Telmisartans 30 Minuten nach dem Beginn eines Auflösungstests bei pH 6,8 in dem Auflösungstest der Japanischen Pharmacopeia gelöst sind.

14. Verfahren zur Herstellung einer pharmazeutischen Tablette, umfassend eine erste Schicht, enthaltend Telmisartan, und eine zweite Schicht, enthaltend Hydrochlorothiazid und Amlodipin, und 10-60% Mannitol, 10-50% mikrokristalline Cellulose und 5-25% Maisstärke.

## Revendications

1. Comprimé pharmaceutique ayant une première couche comprenant du telmisartan, et une seconde couche comprenant de l'hydrochlorothiazide et de l'amlodipine, dans lequel la seconde couche comprend
10 à 60 % de mannitol,
10 à 50 % de cellulose microcristalline et
5 à 25 % d'amidon de maïs.

2. Comprimé pharmaceutique selon la revendication 1, dans lequel la seconde couche comprend 20 à 50 % de mannitol.

3. Comprimé pharmaceutique selon la revendication 2, dans lequel la seconde couche de comprimé comprend 20 à 40 % de cellulose microcristalline.

4. Comprimé pharmaceutique selon la revendication 1, dans lequel la seconde couche de comprimé comprend 10 à 20 % d'amidon de maïs.

5. Comprimé pharmaceutique selon la revendication 2, dans lequel la seconde couche de comprimé comprend 10 à 20 % d'amidon de maïs.

6. Comprimé pharmaceutique selon la revendication 3, dans lequel la seconde couche de comprimé comprend 10 à 20 % d'amidon de maïs.

7. Comprimé pharmaceutique selon la revendication 1, dans lequel plus de 50 % du telmisartan est dissous 60 minutes après le début d'un test de dissolution à pH 1,2 dans le test de dissolution de la pharmacopée japonaise, et
plus de 70 % du telmisartan est dissous 30 minutes après le début d'un test de dissolution à pH 6,8 dans le test de dissolution de la pharmacopée japonaise.

8. Comprimé pharmaceutique selon la revendication 2, dans lequel plus de 50 % du telmisartan est dissous 60 minutes après le début d'un test de dissolution à pH 1,2 dans le test de dissolution de la pharmacopée japonaise, et
plus de 70 % du telmisartan est dissous 30 minutes après le début d'un test de dissolution à pH 6,8 dans le test de dissolution de la pharmacopée japonaise.

9. Comprimé pharmaceutique selon la revendication 3, dans lequel plus de 50 % du telmisartan est dissous 60 minutes après le début d'un test de dissolution à pH 1,2 dans le test de dissolution de la pharmacopée japonaise, et
plus de 70 % du telmisartan est dissous 30 minutes après le début d'un test de dissolution à pH 6,8 dans le test de dissolution de la pharmacopée japonaise.

10. Comprimé pharmaceutique selon la revendication 3, dans lequel plus de 50 % du telmisartan est dissous 60 minutes après le début d'un test de dissolution à pH 1,2 dans le test de dissolution de la pharmacopée japonaise, et
plus de 70 % du telmisartan est dissous 30 minutes après le début d'un test de dissolution à pH 6,8 dans le test de dissolution de la pharmacopée japonaise.

11. Comprimé pharmaceutique selon la revendication 1, dans lequel
plus de 70 % du telmisartan est dissous 60 minutes après le début d'un test de dissolution à pH 1,2 dans le test de dissolution de la pharmacopée japonaise, et
plus de 90 % du telmisartan est dissous 30 minutes après le début d'un test de dissolution à pH 6,8 dans le test de dissolution de la pharmacopée japonaise.

12. Comprimé pharmaceutique selon la revendication 2, dans lequel
plus de 70 % du telmisartan est dissous 60 minutes après le début d'un test de dissolution à pH 1,2 dans le test de dissolution de la pharmacopée japonaise, et
plus de 90 % du telmisartan est dissous 30 minutes après le début d'un test de dissolution à pH 6,8 dans le test de dissolution de la pharmacopée japonaise.

13. Comprimé pharmaceutique selon la revendication 3, dans lequel
plus de 70 % du telmisartan est dissous 60 minutes après le début d'un test de dissolution à pH 1,2 dans le test de dissolution de la pharmacopée japonaise, et
plus de 90 % du telmisartan est dissous 30 minutes après le début d'un test de dissolution à pH 6,8 dans le test de dissolution de la pharmacopée japonaise.

14. Méthode de production d'un comprimé pharmaceutique comprenant une première couche contenant du telmisartan, et une seconde couche contenant de l'hydrochlorothiazide et de l'amlodipine, et 10 à 60 % de mannitol, 10 à 50 % de cellulose microcristalline et 5 à 25 % d'amidon de maïs.
